# EUROPEAN PATENT APPLICATION

(11) **EP 0 930 067 A2**
(43) Date of publication of application: **21.07.1999**
(21) Application number: 98310351.6
(22) Date of filing: 16.12.1998
(51) Int. Cl.: A61K 31/40, A61K 31/445

(54) **MMP inhibitors for the treatment of ocular angiogenesis**

(30) Priority: 19.12.1997 US 68261 P
(71) Applicant: Pfizer Products Inc., Groton, Connecticut 06340 (US)
(72) Inventor: Doherty, Niall Stephen, Stonington, Connecticut (US)
(74) Representative: McMunn, Watson Palmer

(57) **Abstract**

The present invention relates to the use of matrix metalloproteinase inhibitors, preferably those which display specificity for matrix metalloproteinases-2 or 9, in the treatment or prevention of ocular angiogenesis.

## Description

### BACKGROUND OF THE INVENTION

The present invention relates to the use of matrix metalloproteinase inhibitors, preferably those which display specificity for matrix metalloproteinases-2 or 9 in the treatment or prevention of ocular angiogenesis, i.e macular degeneration and diabetic retinopathy.

Aberrant angiogenesis occurs in several different disease areas including cancer, rheumatoid arthritis and psoriasis. Angiogenesis also occurs in various eye diseases such as diabetic retinopathy and age related macular degeneration (ARMD). Taken together 2.4 million individuals are affected by these two ocular indications (0.7 million for proliferative diabetic retinopathy and 1.7 million for angiogenic ARMD). Laser photocoagulation is a highly effective therapy for proliferative diabetic retinopathy but has side effects, primarily a variable loss of peripheral and night vision. Currently there is no therapy capable of preventing or adequately treating ARMD.

Complications of diabetes is the major cause of blindness in patents under 50 years of age. In diabetic retinopathy, new blood vessels overlying the retina develop, exhibit increased vascular permeability, micro-anurysms and hemorrhage resulting in damage to the neural retina. The current therapy for diabetic retinopathy is to photocoagulate the retina outside the macula so that the blood vessels are destroyed and cannot further proliferate. This treatment has a success rate of approximately 95% but has side effects, primarily a variable loss of peripheral and night vision. The incidence of diabetes related ocular angiogenesis is 25,000 cases/year.

Age-related macular degeneration (ARMD) is the leading cause of legal blindness and low vision in the elderly. This disorder represents an advanced and pathologic stage of the aging process that occurs in all eyes. Age-related macular degeneration exists in a "dry" or "wet" form. The "dry" or atrophic type causes gradual deterioration of vision over 10 to 20 years. The "wet" or exudative type causes a rapid and widespread loss of central vision due to leakage of blood or fluid from abnormal, newly formed blood vessels (i.e., choroidal neovascular membranes) proliferating beneath the macula. Since this process occurs in the macula there is a major impact on acuity and central vision. Unlike diabetes related blindness, patients with ARMD are frequently healthy in other respects and the loss of vision can convert an active independent individual into one totally dependent on others. In ARMD, one eye usually shows symptoms first and patients are faced with several years of anguish waiting for the likely loss of sight in the other eye. There is no effective way to treat or prevent this condition. Photocoagulation therapy cannot be used since it destroys the photoreceptors and would cause blindness if applied to the macula. A category of ARMD considered to have a high risk of developing the exudative type is distinguishable by clinical examination. Since the prevalence of this category of ARMD doubles with every decade after 50, and the aging of the populations of the developed countries, the suffering and health care costs of this condition are likely to grow dramatically. In a US population study, in males over the age of thirty, the prevalence of exudative disease was 0.5% but the prevalence of the high risk condition, (prime candidates for prophylactic therapy) was 9% (Bessler et al, Arch Opthalmol, 107, 847(1989). In the over 70 age group prevalence of the high risk condition rose to 26%. This is a major unmet medical need.

The present inventor has now discovered that matrix metalloproteinase inhibitors, preferably those which display specificity for matrix metalloproteinases-2 or 9, are useful for the treatment or prevention of ocular angiogenesis, i.e. macular degeneration and diabetic retinopathy.

### SUMMARY OF THE INVENTION

The present invention relates to a method of treating or preventing ocular angiogenesis, including macular degeneration and diabetic retinopathy, by inhibiting a matrix metalloproteinase in a mammal, preferably a human, comprising administering to said mammal requiring such treatment or prevention a matrix metalloproteinase inhibiting effective amount of a matrix metalloproteinase inhibitor, or pharmaceutically acceptable salts thereof.

The present invention also relates to a method of treating or preventing ocular angiogenesis, including macular degeneration and diabetic retinopathy, in a mammal, preferably a human, comprising administering to said mammal requiring such treatment or prevention a matrix metalloproteinase-2 or matrix metalloproteinase-9 inhibiting effective amount of a matrix metalloproteinase-2 or matrix metalloproteinase-9 inhibitor, or pharmaceutically acceptable salts thereof.

The present invention also relates to a method of treating or preventing ocular angiogenesis, including macular degeneration and diabetic retinopathy, in a mammal, preferably a human, comprising administering to said mammal requiring such treatment or prevention a matrix metalloproteinase-2 selective or matrix metalloproteinase-9 selective inhibiting effective amount of a matrix metalloproteinase-2 selective or matrix metalloproteinase-9 selective inhibitor, or pharmaceutically acceptable salts thereof.

The present invention also relates to a method of treating or preventing ocular angiogenesis, including macular degeneration and diabetic retinopathy, in a mammal, preferably a human, comprising administering to said mammal requiring such treatment or prevention a matrix metalloproteinase inhibiting effective amount of a compound selected from the group consisting of:
3-[[4-(4-fluoro-phenoxy)-benzenesulfonyl]-(1-hydroxycarbamoyl-cyclopentyl)-amino]-propionic acid;
4-[4-(4-fluoro-phenoxy)-benzenesulfonylamino]-tetrahydro-pyran-4-carboxylic acid hydroxyamide;
4-[4-(4-chloro-phenoxy)-benzenesulfonylmethyl]-tetrahydro-pyran-4-carboxylic acid hydroxyamide;
3-[[4-(4-fluoro-phenoxy)-benzenesulfonyl]-(1-hydroxycarbamoyl-cyclobutyl)-amino]-propionic acid;
4-(4'-chloro-biphenyl-4-yl)-2-[2-(1,3-dioxo-1,3-dihydro-isoindol-2-yl)-ethyl]-4-oxo-butyric acid;
{1-[4-(4-fluoro-benzyloxy)-benzenesulfonyl]-2-hydroxycarbamoyl-piperidin-3-yl}-carbamic acid isopropyl ester;
2-[4-(4-fluoro-phenoxy)-benzenesulfonylamino]-N-hydroxy-2-methyl-propionamide;
3-[4-(4-fluoro-phenoxy)-benzenesulfonyl]-2,N-dihydroxy-propionamide;
3-(4-phenoxy-benzenesulfonyl)-7-oxa-bicyclo[2.2.1]heptane-2-carboxylic acid hydroxyamide;
(4-benzyl-benzyl)-[2-(2,2-dimethyl-1-methylcarbamoyl-propylcarbamoyl)-4-(4'-fluoro-biphenyl-4-yl)-butyl]-phosphinic acid ;
2-amino-3-[4-(4-fluoro-phenoxy)-benzenesulfonyl]-N-hydroxy-propionamide; and
N-hydroxy-2-(4-phenyl-piperidine-1-sulfonyl)-acetamide.

The present invention also relates to an intraorbital pharmaceutical composition for treating or preventing ocular angiogenesis, including macular degeneration and diabetic retinopathy, in a mammal, comprising an amount of a matrix metalloproteinase inhibitor that is effective in treating or preventing ocular angiogenesis, including macular degeneration and diabetic retinopathy, and a pharmaceutically acceptable carrier.

### DETAILED DESCRIPTION OF THE INVENTION

Matrix metalloproteinase inhibitors, induding MMP-2 or MMP-9 selective inhibitors, can be prepared according to methods well known to those of ordinary skill in the art. Specifically, matrix metalloproteinase inhibitors with broad activity and their methods of preparation have been described in PCT publications WO 96/33172, published October 24, 1996, and WO 96/27583, published March 7, 1996; PCT Application No. PCT/US95/07166, filed June 07, 1995 (which corresponds to United States Patent Application No. 60/017,850, filed May 20, 1996); PCT Application No. PCT/IB95/00427, filed June 6, 1995; European Patent Application No. 0818442; published January 14, 1998; PCT published Application No. WO 98/07697; published PCT Application No. WO 98/03516; PCT published Application No. WO 98/34918; PCT published Application No. WO 98/34915; PCT published Application No. WO 98/33768; PCT published Application No. WO 98/30566. European Patent Application 606,046, published July 13; 1994; PCT publication WO 90/05719, published May 31, 1990; and European Patent Publication 780,386, published June 25, 1997. All of the foregoing references are incorporated herein by reference in their entirety.

Matrix metallogproteinase -2 or -9 selective inhibitors refer to compounds that demonstrate a selectivity for the MMP-2 or -9 metalloproteinase over MMP-1 metalloproteinase or other MMPs.

The ability of matrix metalloproteinase inhibitors or or their pharmaceutically acceptable salts (hereinafter also referred to as the inhibitors of the present invention) to inhibit matrix metalloproteinases or the production of tumor necrosis factor (TNF) and, consequently, demonstrate their effectiveness for treating macular degeneration is shown by the following in vitro assay tests.

### Biological Assay

### Inhibition of Human Collagenase (MMP-1)

Human recombinant collagenase is activated with trypsin using the following ratio: 10 mg trypsin per 100 mg of collagenase. The trypsin and collagenase are incubated at room temperature for 10 minutes then a five fold excess (50 mg/10 mg trypsin) of soybean trypsin inhibitor is added.

10 mM stock solutions of inhibitors are made up in dimethyl sulfoxide and then diluted using the following Scheme:
10 mM → 120 µM → 12 µM → 1.2 µM → 0.12 µM

Twenty-five microliters of each concentration is then added in triplicate to appropriate wells of a 96 well microfluor plate. The final concentration of inhibitor will be a 1:4 dilution after addition of enzyme and substrate. Positive controls (enzyme, no inhibitor) are set up in wells D1-D6 and blanks (no enzyme, no inhibitors) are set in wells D7-D12.

Collagenase is diluted to 400 ng/ml and 25 ml is then added to appropriate wells of the microfluor plate. Final concentration of collagenase in the assay is 100 ng/ml.

Substrate (DNP-Pro-Cha-Gly-Cys(Me)-His-Ala-Lys(NMA)-NH₂) is made as a 5 mM stock in dimethyl sulfoxide and then diluted to 20 mM in assay buffer. The assay is initiated by the addition of 50 ml substrate per well of the microfluor plate to give a final concentration of 10 mM.

Fluorescence readings (360 nM excitation, 460 nm emission) were taken at time 0 and then at 20 minute intervals. The assay is conducted at room temperature with a typical assay bme of 3 hours.

Fluorescence vs time is then plotted for both the blank and collagenase containing samples (data from triplicate determinations is averaged). A time point that provides a good signal (the blank) and that is on a linear part of the curve (usually around 120 minutes) is chosen to determine IC₅₀ values. The zero time is used as a blank for each compound at each concentration and these values are subtracted from the 120 minute data. Data is plotted as inhibitor concentration vs % control (inhibitor fluorescence divided by fluorescence of collagenase alone x 100). IC₅₀'s are determined from the concentration of inhibitor that gives a signal that is 50% of the control.

If IC₅₀'s are reported to be <0.03 mM then the inhibitors are assayed at concentrations of 0.3 mM, 0.03 mM, 0.03 mM and 0.003 mM.

### Inhibition of Gelatinase (MMP-2)

Inhibition of gelatinase activity is assayed using the Dnp-Pro-Cha-Gly-Cys(Me)-His-Ala-Lys(NMA)-NH₂ substrate (10 mM) under the same conditions as inhibition of human collagenase (MMP-1).

72kD gelatinase is activated with 1 mM APMA (p-aminophenyl mercuric acetate) for 15 hours at 4°C and is diluted to give a final concentration in the assay of 100 mg/ml. Inhibitors are diluted as for inhibition of human collagenase (MMP-1) to give final concentrations in the assay of 30 mM, 3 mM, 0.3 mM and 0.03 mM. Each concentration is done in triplicate.

Fluorescence readings (360 nm excitation, 460 emission) are taken at time zero and then at 20 minutes intervals for 4 hours.

IC₅₀'s are determined as per inhibition of human collagenase (MMP-1). If IC₅₀'s are reported to be less than 0.03 mM, then the inhibitors are assayed at final concentrations of 0.3 mM, 0.03 mM, 0.003 mM and 0.003 mM.

### Inhibition of Stromelysin Activity (MMP-3)

Inhibition of stromelysin activity is based on a modified spectrophotometric assay described by Weingarten and Feder (Weingarten, H. and Feder, J., Spectrophotometric Assay for Vertebrate Collagenase, Anal. Biochem. 147, 437-440 (1985)). Hydrolysis of the thio peptolide substrate [Ac-Pro-Leu-Gly-SCH[CH₂CH(CH₃)₂]CO-Leu-Gly-OC₂H₅] yields a mercaptan fragment that can be monitored in the presence of Ellman's reagent.

Human recombinant prostromelysin is activated with trypsin using a ratio of 1 ml of a 10 mg/ml trypsin stock per 26 mg of stromelysin. The trypsin and stromelysin are incubated at 37°C for 15 minutes followed by 10 ml of 10 mg/ml soybean trypsin inhibitor for 10 minutes at 37°C for 10 minutes at 37°C to quench trypsin activity.

Assays are conducted in a total volume of 250 ml of assay buffer (200 mM sodium chloride, 50 mM MES, and 10 mM calcium chloride, pH 6.0) in 96-well microliter plates. Activated stromelysin is diluted in assay buffer to 25 mg/ml. Ellman's reagent (3-Carboxy-4-nitrophenyl disulfide) is made as a 1M stock in dimethyl formamide and diluted to 5 mM in assay buffer with 50 ml per well yielding at 1 mM final concentration.

10 mM stock solutions of inhibitors are made in dimethyl sulfoxide and diluted serially in assay buffer such that addition of 50 mL to the appropriate wells yields final concentrations of 3 mM, 0.3 mM, 0.003 mM, and 0.0003 mM. All conditions are completed in triplicate.

A 300 mM dimethyl sulfoxide stock solution of the peptide substrate is diluted to 15 mM in assay buffer and the assay is initiated by addition of 50 ml to each well to give a final concentration of 3 mM substrate. Blanks consist of the peptide substrate and Ellman's reagent without the enzyme. Product formation was monitored at 405 nm with a Molecular Devices UVmax plate reader.

IC₅₀ values were determined in the same manner as for collagenase.

### Inhibition of Human 92 kD Gelatinase (MMP-9)

Inhibition of gelatinase (MMP-9) activity is assayed using the Mca-Pro-Leu-Gly-Leu-Dpa-Ala-Arg-NH₂ substrate (10 mM) under similar conditions as described above for the inhibition of human collagenase (MMP-1).

Human recombinant 92 kD gelatinase (MMP-9, gelatinase B) is activated for 2 hours with 1mM p-aminophenyl-mercuric acetate (from a freshly prepared 100 mM stock in 0.2 N NaOH) at 37 C.

10 mM dimethylsulfoxide stock solutions of inhibitors are diluted serially in assay buffer (50 mM TRIS, pH 7.5, 200 mM NaCI, 5 mM CaCl₂, 20 µM ZnCl₂, 0.02% BRIJ-35 (vol./vol.)) using the following scheme:
10 mM--→ 120 µM---→ 12 µM---→ 1.2 µM---→ 0.12 µM

Further dilutions are made as necessary following this same scheme. A minimum of four inhibitor concentrations for each compound are performed in each assay. 25 µL of each concentration is then added to triplicate wells of a black 96 well U-bottomed microfluor plate. As the final assay volume is 100 µL, final concentrations of inhibitor are the result of a further 1:4 dilution (i.e. 30 µM ---→ 3 µM ---→ 0.3 µM ---→ 0.03 µM, etc.). A blank (no enzyme, no inhibitor) and a positive enzyme control (with enzyme, no inhibitor) are also prepared in triplicate.

Activated enzyme is diluted to 100 ng/mL in assay buffer, 25 µL per well is added to appropriate wells of the microplate. Final enzyme concentration in the assay is 25 ng/mL (0.27 nM).

A five mM dimethylsulfoxide stock solution of substrate (Mca-Pro-Leu-Gly-Leu-Dpa-Ala-Arg-NH₂) is diluted in assay buffer to 20 µM. The assay is initiated by addition of 50 µL of diluted substrate yielding a final assay concentration of 10 µM substrate. A 0 time fluorescence reading (320 excitation; 390 emission) is immediately taken and subsequent readings are taken every fifteen minutes at room temperature with a PerSeptive Biosystems CytoFluor Multi-Well Plate Reader with the gain at 90 units.

The average value of fluorescence of the enzyme and blank are plotted versus time An early time point on the linear part of this curve is chosen for IC₅₀ determinations. The 0 time point for each compound at each dilution is subtracted from the latter time point and the data then expressed as percent of enzyme control (inhibitor fluorescence divided by fluorescence of positive enzyme control x 100). Data is plotted as inhibitor concentration versus percent of enzyme control. IC₅₀'s are defined as the concentration of inhibitor that gives a signal that is 50% of the positive enzyme control.

### Inhibition of MMP-13

Human recombinant MMP-13 is activated with 2mM APMA (p-aminophenyl mercuric acetate) for 1.5 hours, at 37°C and is diluted to 400 mg/ml in assay buffer (50 mM Tris, pH 7.5, 200 mM sodium chloride, 5mM calcium chloride, 20mM zinc chloride, 0.02% brij). Twenty-five microliters of diluted enzyme is added per well of a 96 well microfluor plate. The enzyme is then diluted in a 1:4 ratio in the assay by the addition of inhibitor and substrate to give a final concentration in the assay of 100 mg/ml.

10 mM stock solutions of inhibitors are made up in dimethyl sulfoxide and then diluted in assay buffer as per the inhibitor dilution scheme for inhibition of human collagenase (MMP-1). Twenty-five microliters of each concentration is added in triplicate to the microfluor plate. The final concentrations in the assay are 30 mM, 3mM, 0.3 mM, and 0.03 mM.

Substrate (Dnp-Pro-Cha-Gly-Cys(Me)-His-Ala-Lys(NMA)-NH₂) is prepared as for inhibition of human collagenase (MMP-1) and 50 ml is added to each well to give a final assay concentration of 10 mM. Fluorescence readings (360 nM excitation; 450 emission) are taken at time 0 and every 5 minutes for 1 hour.

Positive controls consist of enzyme and substrate with no inhibitor and blanks consist of substrate only.

IC₅₀'s are determined as per inhibition of human collagenase (MMP-1). If IC₅₀'s are reported to be less than 0.03 mM, inhibitors are then assayed at final concentrations of 0.3 mM, 0.03 mM, 0.003 mM and 0.0003 mM.

All of the compounds of the invention that were tested in the Inhibition of MMP-13 assay had IC₅₀'s of less than 50nm.

### Inhibition of TNF Production

The ability of the compounds or the pharmaceutically acceptable salts thereof to inhibit the production of TNF and, consequently, demonstrate their effectiveness for treating diseases involving the production of TNF is shown by the following in vitro assay:

Human mononuclear cells were isolated from anticoagulated human blood using a one-step Ficoll-hypaque separation technique. (2) The mononuclear cells were washed three times in Hanks balanced salt solution (HBSS) with divalent cations and resuspended to a density of 2 x 10⁶ /ml in HBSS containing 1% BSA. Differential counts determined using the Abbott Cell Dyn 3500 analyzer indicated that monocytes ranged from 17 to 24% of the total cells in these preparations.

180m of the cell suspension was aliquoted into flat bottom 96 well plates (Costar). Additions of compounds and LPS (100ng/ml final concentration) gave a final volume of 200ml. All conditions were performed in triplicate. After a four hour incubation at 37°C in an humidified CO₂ incubator, plates were removed and centrifuged (10 minutes at approximately 250 x g) and the supematants removed and assayed for TNFα using the R&D ELISA Kit®.

### Inhibition of Angiogenesis

The activity of MMP inhibitors, preferably MMP-2 or MMP-9 inhibitors, for inhibiting angiogenesis can be determined according to the following *in vivo* rat model. A 10µg pellet containing an angiogenic material, such as vascular endothelial growth factor (VEGF) and a matrix metalloproteinase inhibitor or placebo, is implanted intraorbitally. After one week the eyes are removed and the degree of inhibition of angiogenesis is determined qualitatively. Inhibitors with IC₅₀'s of less than 10nm reduced angiogenesis. Table 1 demonstrates that MMP inhibitors are useful in treating angiogenesis.

**Table 1**

| Compound | Corneal Neovascularization (#Positive Response/#Response) | % Response |
|---|---|---|
| Hydron + PBS | 0/3 | 0 |
| basic fibroblast growth factor | 3/3 | 100 |
| VEGF | 4/4 | 100 |
| 3-[[4-(4-Fluoro-phenoxy)-benzenesulfonyl]-(1-hydroxycarbamoyl-cyclopentyl)-amino]-propionic acid | | |
| 10ng + VEGF | 1/4 | 25 |
| 25ng + VEGF | 1/6 | 17 |
| 50ng + VEGF | 0/5 | 0 |
| 100ng + VEGF | 0/5 | 0 |
| 2-[4-(4-Fluoro-phenoxy)-benzenesulfonylamino]-N-hydroxy-2-methyl-propionamide | | |
| 10ng + VEGF | 1/5 | 20 |
| 25ng + VEGF | 1/6 | 17 |
| 50ng + VEGF | 0/5 | 0 |
| 100ng + VEGF | 0/5 | 0 |
| 3-(4-Phenoxy-benzenesulfonyl)-7-oxa-bicyclo[2.2.1]heptane-2-carboxylic acid hydroxyamide | | |
| 10ng + VEGF | 4/4 | 25 |
| 25ng + VEGF | 3/5 | 60 |
| 50ng + VEGF | 2/5 | 40 |
| 100ng + VEGF | 1/5 | 20 |

The compositions of the present invention may be formulated in a conventional manner using one or more pharmaceutically acceptable carriers. Thus, the active compounds of the invention may be formulated for oral, intraorbital, buccal, intranasal, parenteral (e.g., intravenous, intramuscular or subcutaneous) or rectal administration or in a form suitable for administration by inhalation or insufflation.

The active compounds can be administered in a wide variety of different dosage forms, in general, the therapeutically effective compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelation and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof. In the case of animals, they are advantageously contained in an animal feed or drinking water in a concentration of 5-5000 ppm, preferably 25 to 500 ppm.

For intraorbital administration a sterile injectable solution of the active ingredient is usually prepared. Solutions of a therapeutic compound of the present invention in an aqueous solution or suspension (particle size less than 10 micron) may be employed. The aqueous solutions should be suitably adjusted and buffered, preferably at a pH between 5 and 8, if necessary and the liquid diluent first rendered isotonic. Small amounts of polymers can be added to increase viscosity or for sustained release (such as cellulosic polymers, Dextran, polyethylene glycol, or alginic acid). These solutions are suitable for intraorbital injection purposes. The preparation of all these solutions under sterile conditions is readily accomplished by standard pharmaceutical techniques well known to those skilled in the art. In the case of animals, compounds can be administered intraorbitally at dosage levels of about 0.1 to 50 mg/kg/day, advantageously 0.2 to 10 mg/kg/day given in a single dose or up to 3 divided doses.

For parenteral administration (intramuscular, intraperitoneal, subcutaneous and intravenous use) a sterile injectable solution of the active ingredient is usually prepared. Solutions of a therapeutic compound of the present invention in either sesame or peanut oil or in aqueous propylene glycol may be employed. The aqueous solutions should be suitably adjusted and buffered, preferably at a pH of greater than 8, if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art. In the case of animals, compounds can be administered intramuscularly or subcutaneously at dosage levels of about 0.1 to 50 mg/kg/day, advantageously 0.2 to 10 mg/kg/day given in a single dose or up to 3 divided doses.

The active compounds may also be formulated in rectal compositions such as suppositories or retention enemas, e.g., containing conventional suppository bases such as cocoa butter or other glycerides.

For intranasal administration or administration by inhalation, the active compounds are conveniently delivered in the form of a solution or suspension from a pump spray container that is squeezed or pumped by the patient or as an aerosol spray presentation from a pressurized container or a nebulizer, with the use of a suitable propellant e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

## Claims

1. Use of a matrix metalloprotienase inhibitor, or pharmaceutically acceptable salts thereof in the preparation of a medicament for the treatment or prevention of ocular angiogenesis in a mammal, preferably a human being.

2. Use according to Claim 1 wherein the matrix metalloprotienase inhibitor is a matrix metalloproteinase-2-inhibitor or matrix metalloproteinase-3 inhibitor.

3. Use according to Claim 2 wherein the matrix metalloproteinase inhibitor is a matrix metalloproteinase-2 selective or matrix metalloproteinase-9 selective inhibitor.

4. Use according to any of the preceeding claims wherein the matrix metalloproteinase inhibitor is selected from the group consisting essentially of:
3-[[4-(4-fluoro-phenoxy)-benzenesulfonyl]-(1-hydroxycarbamoyl-cyclopentyl)-amino]-propionic acid;
4-[4-(4-fluoro-phenoxy)-benzenesulfonylamino]-tetrahydro-pyran-4-carboxylic acid hydroxyamide;
4-[4-(4-chloro-phenoxy)-benzenesulfonylmethyl]-tetrahydro-pyran-4-carboxylic acid hydroxyamide;
3-[[4-(4-fluoro-phenoxy)-benzenesulfonyl]-(1-hydroxycarbamoyl-cyclobutyl)-amino]-propionic acid;
4-(4'chloro-biphenyl-4-yl)-2-[2-(1,3-dioxo-1,3-dihydro-isoindol-2-yl)-ethyl]-4-oxo-butyric acid;
{1-[4-(4-fluoro-benzyloxy)-benzenesulfonyl]-2-hydroxycarbamoyl-piperidin-3-yl}-carbamic acid isopropyl ester;
2-[4-(4-fluoro-phenoxy)-benzenesulfonylamino]-N-hydroxy-2-methyl-propionamide;
3-[4-(4-fluoro-phenoxy)-benzenesulfonyl]-2,N-dihydroxy-propionamide;
3-(4-phenoxy-benzenesulfonyl)-7-oxa-bicyclo[2.2.1]heptane-2-carboxylic acid hydroxyamide;
(4-benzyl-benzyl)-[2-(2,2-dimethyl-1-methylcarbamoyl-propylcarbamoyl)-4-(4'-fluoro-biphenyl-4-yl)-butyl]-phosphinic acid ;
2-amino-3-[4-(4-fluoro-phenoxy)-benzenesulfonyl]-N-hydroxy-propionamide; and
N-hydroxy-2-(4-phenyl-piperidine-1-sulfonyl)-acetamide.

5. A pharmaceutical composition for treating or preventing ocular angiogenesis in a mammal, comprising a matrix metalloproteinase inhibitor according to any of the preceeding claims and a pharmaceutically acceptable carrier.
